# EUROPEAN PATENT SPECIFICATION

(11) **EP 3 939 636 B1**
(45) Date of publication and mention of the grant of the patent: **06.05.2026**
(21) Application number: 20772978.1
(22) Date of filing: 13.03.2020
(51) Int. Cl.: A61M 5/20, A61M 5/32

(54) **ASSISTANCE INSTRUMENT FOR SYRINGE**
ASSISTENZINSTRUMENT FÜR SPRITZE
INSTRUMENT D'ASSISTANCE POUR SERINGUE

(30) Priority: 15.03.2019 JP 2019048906
(43) Date of publication of application: 19.01.2022
(73) Proprietor: Taisei Kako Co., Ltd., Kita-ku Osaka-shi Osaka 531-0072 (JP)
(72) Inventor: SOMEKAWA, Tsuyoshi, Ibaraki-shi, Osaka 567-0054 (JP)
(74) Representative: Stolmár & Partner Patentanwälte PartG mbB
(86) International application number: PCT/JP2020/011221
(87) International publication number: WO 2020/189573

(56) References cited:
- WO-A1-00/24441
- WO-A1-02/083205
- WO-A1-2017/179638
- JP-A- 2005 500 871
- JP-A- 2017 189 515
- JP-A- 2017 189 515
- JP-A- H08 505 543
- US-A- 5 681 291

## Description

### CROSS-REFERENCE TO RELATED APPLICATION

This application claims priority to Japanese Patent Application No. 2019 - 048906.

### FIELD

The present invention relates to a syringe auxiliary tool for ease of injection.

### BACKGROUND

There was conventionally a sole use of a syringe for injection, but in this case, it was difficult to adjust the depth of the injection needle insertion into a living body. Therefore, various syringe auxiliary tools having an injection needle capable of being easily inserted into a living body at an appropriate depth to thereby enable easy injection.

For example, a syringe auxiliary tool (or a drug delivery device) disclosed in Patent Literature 1 includes a lower housing that holds a syringe with a syringe needle protruding from a lower end of the lower housing, an upper housing that is externally fitted onto an upper end portion of the lower housing and is configured to be slid toward a lower end of the lower housing for pressing-in of a plunger of the syringe, a needle guard that is capable of advancing toward and retracting from the lower end of the lower housing so as to cover or expose the needle, and a needle guard return element that biases the needle guard toward a needle protruding side.

This drug delivery device is configured to expose the needle by a predetermined length by retracting the needle guard, then, in this state, pierce the needle into a living body, and then push-in the upper housing to thereby perform an injection.

Further, for example, a syringe auxiliary tool (injection device) disclosed in Patent Literature 2 includes a housing that has a distal end configured to be forced against a living body, a moving element disposed in the housing and configured to be capable of holding a hypodermic syringe, and to be attached to the housing slidably in a front-rear direction, a spring that biases the moving element toward a distal end side of the housing, and a locking mechanism capable of restricting the moving element, which has been slid toward the rear end side of the housing, from sliding toward the front side of the housing.

According to this injection device, the moving element before use (before performing an injection), which is in the state of holding the hypodermic syringe, is disposed on the rear end side of the housing in advance, while being restricted from sliding toward the front side by a locking mechanism.

When injecting using the injection device, the distal end of the housing is forced against the living body, and then the restriction of the sliding by the lock mechanism is released. This causes the moving element to slide toward the distal end side of the housing together with the hypodermic syringe under the influence of the biasing force of the spring to thereby allow a hypodermic needle of the hypodermic syringe to be pierced into the living body by a predetermined length.

### CITATION LIST

### Patent Literature

Patent Literature 1: JP 6219353 B
Patent Literature 2: JP 2016-525417 T

### SUMMARY

### Technical Problem

Meanwhile, the conventional drug delivery device as aforementioned is configured to keep the needle exposed by the predetermined length, and thus may cause the needle, which has been accidentally pierced into the living body , not to be instantly pulled out of the living body. A similar problem may also occur with the injection device as aforementioned. Accordingly, there is a demand for enhanced safety during piercing of an injection needle in the conventional drug delivery device and the syringe auxiliary tool, such as the injection device as aforementioned.

In view of the aforementioned circumstances, it is an object of the present invention to provide a syringe auxiliary tool capable of enhancing the safety during piercing an injection needle.

### Solution to Problem

The syringe auxiliary tool of the present invention includes:
a housing that has a contact surface that comes into contact with a living body and houses a syringe with an injection needle directed forward;
a moving part that engages with the syringe housed in the housing and attached to the housing slidably in a front-rear direction, the moving part being capable of changing its position, in association with the sliding, between an advanced position at which the injection needle of the syringe is located forward of the contact surface and a retracted position at which the injection needle is located rearward of the contact surface;
a biasing means that biases the moving part in a retracting direction from the advanced position toward the retracted position; and
an operation member that is configured to slide the moving part in a forward direction from the retracted position toward the advanced position.

The syringe auxiliary tool of the present invention further includes:
a connecting part that connects the moving part with the operation member and slides the moving part in association with a movement of the operation member; and
a restriction structure that restricts the moving part from sliding from the advanced position toward the retracted position in a state where the moving part is located at the advanced position, wherein
the operation member is configured to be separated from the connecting part in a state where the moving part is located at the advanced position.

In the syringe auxiliary tool of the present invention,
the operation member is capable of switching between a first position before it slides the moving part to the advanced position and a second position after it has slid the moving part to the advanced position,
the operation member includes a pressing part that presses the connecting part to slide the moving part to the advanced position and a blocking part that blocks the movement of the connecting part toward a direction in which the connecting part is pressed by the pressing part,
the connecting part includes a pressing operation engaging part that engages with the pressing part and a block engaging part that engages with the blocking part, and
the syringe auxiliary tool is configured such that:
   the pressing part and the pressing operation engaging part are in engagement with each other while the blocking part and the block engaging part are out of engagement with each other when the operation member is located at the first position; and
   when the operation member is located at the second position, the pressing part and the pressing operation engaging part are out of engagement with each other, the operation member is separated from the connecting part, the restriction by the restriction structure is released, and the blocking part and the block engaging part engage with each other when the moving part and the connecting part return from the advanced position to the retracted position by a biasing force of the biasing means.

### BRIEF DESCRIPTION OF DRAWINGS

Fig. 1 is a perspective view of a syringe auxiliary tool according to an embodiment of the present invention.
Fig. 2 is a plan view of the syringe auxiliary tool according to the embodiment.
Fig. 3 is an exploded perspective view of the syringe auxiliary tool according to the embodiment.
Fig. 4 is a cross-sectional view taken along a section line IV-VI in Fig. 2.
Fig. 5 is a plan view showing inside of a housing of the syringe auxiliary tool according to the embodiment.
Fig. 6 is an explanatory view showing an operation for removing a cap from a syringe in the syringe auxiliary tool according to the embodiment in a state before the syringe with the cap attached thereto is placed into the housing.
Fig. 7 is an explanatory view showing an operation for removing the cap from the syringe in the syringe auxiliary tool according to the embodiment, in which the syringe with the cap attached thereto is placed into the housing and a separation structure is interposed between the cap and the syringe.
Fig. 8 is an explanatory view showing an operation of removing the cap from the syringe in the syringe auxiliary tool according to the embodiment, in which the cap and the syringe are separated from each other by the separation structure.
Fig. 9 is an explanatory view of an injection needle piercing operation in the syringe auxiliary tool according to the embodiment in a state before the syringe is moved forward.
Fig. 10 is an explanatory view of the injection needle piercing operation in the syringe auxiliary tool according to the embodiment in a state where the syringe has been moved forward.
Fig. 11 is an explanatory view of an operation of pulling out the injection needle from a living body in the syringe auxiliary tool according to the embodiment in a state where a plunger of the syringe has been moved forward.
Fig. 12 is an explanatory view showing an operation of pulling out the injection needle from the living body in the syringe auxiliary tool according to the embodiment in a state where the syringe has been moved rearward.

### DESCRIPTION OF EMBODIMENTS

Hereinafter, a syringe auxiliary tool according to an embodiment of the present invention will be described with reference to the attached drawings. The syringe auxiliary tool according to this embodiment is configured to enable the injection simply and safely.

The injection using the syringe auxiliary tool may be made by a patient himself or herself, or may be performed by another person such as a doctor for a patient.

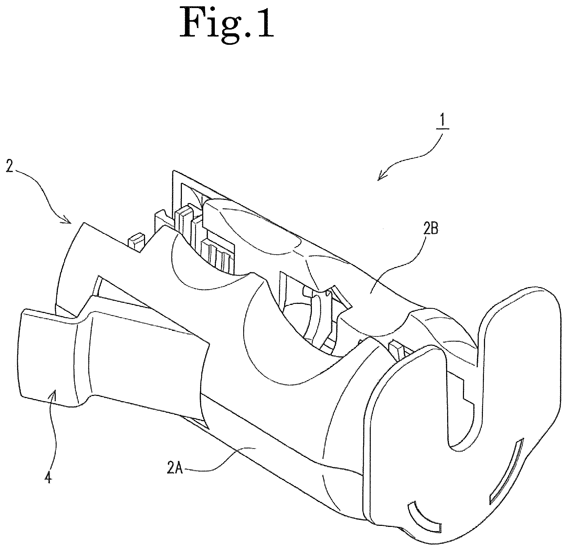

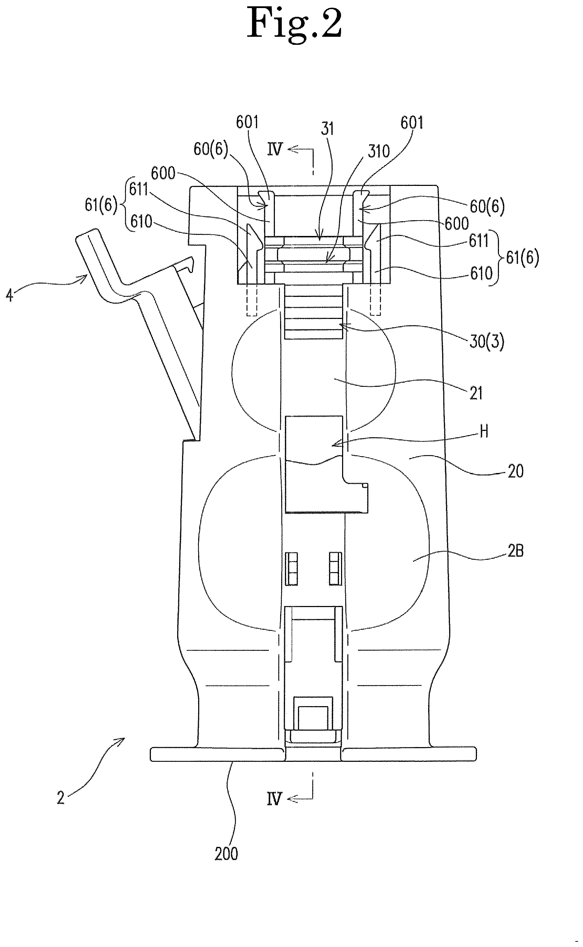

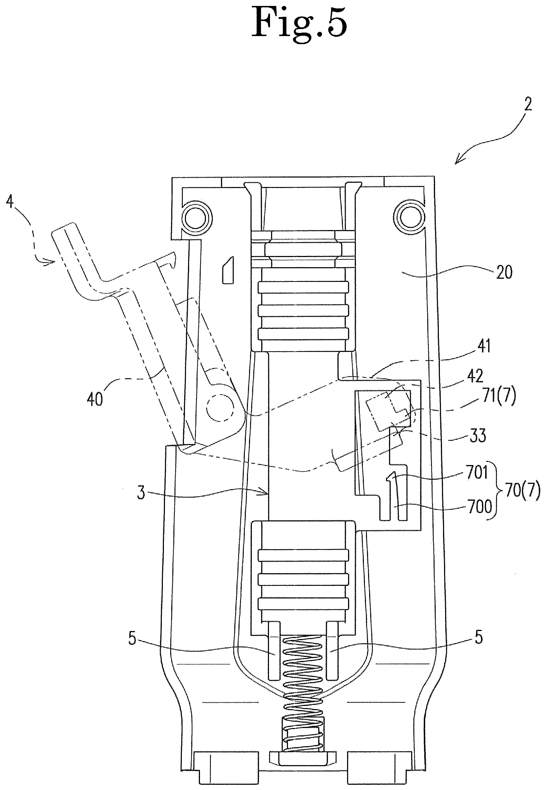

A syringe auxiliary tool 1 includes a housing 2 that houses a syringe as shown in Fig. 1, and further includes, as shown in Fig. 5, a moving part 3 configured to slide back and forth in the housing 2 together with the syringe housed in the housing 2, an operation member 4 that is operated by a user to slide the moving part 3, a separation structure 5 designed for a syringe with a cap attached thereto (hereinafter, referred to as a capped syringe) housed in the housing 2 to separate the cap and the syringe from each other, a restriction structure 6 (see Fig. 2) that is capable of restricting the syringe from moving rearward in the state where the injection needle advances from the housing 2, and a blocking structure 7 that is capable of blocking the syringe from moving forward in the state where the injection needle is retracted in the housing 2.

As shown in Fig. 2, the housing 2 includes a housing body 20 having a front end and a rear end, and an opening H continuously extending from the front end to the rear end, a receiving part 21 that receives the capped syringe placed (inserted) into the housing body 20 through the opening H, and a coming-off prevention part 22 that prevents the syringe received by the receiving part 21 from coming off from the opening H.

The housing 2 according to this embodiment is formed by a case part 2A and a cover part 2B that is mounted on the case 2A and united therewith, the case part 2A is configured to receive the moving part 3, the operation member 4, the separation structure 5, and the restriction structure 6, and the cover part 2B forms therein the opening H and the receiving part 21. That is, the case part 2Aforms a bottom part of the housing 2, and the cover part 2B forms a top part of the housing 2.

The housing body 20 has an elongated cylindrical shape and has one end surface (distal end surface) in the longitudinal direction serving as a contact surface 200 that comes into contact with a living body at the time of injection. Further, the opening H formed in the housing body 20 extends straight in the longitudinal direction of the housing body 20, and thus the capped syringe is housed in the housing body 20 with its posture laid sideways, that is, with an axial direction of the syringe corresponding to the longitudinal direction of the housing body 20. Therefore, the syringe housed in the housing 2 slides toward the distal end side in the longitudinal direction of the housing body 20 to thereby cause a part of the injection needle to move forward of the contact surface 200. Of the entire injection needle, a part which is located forward of the contact surface 200 at this time serves as a part to be pierced into a living body.

The following description will be made with a direction in which the front end and the rear end of the housing body 20 are aligned to each other (i.e., a direction in which the syringe slides in this embodiment) referred to as a front-rear direction, a direction in which the opening H opens (i.e., a direction in which the bottom part and the top part of the housing 2 are aligned to each other) as a height direction, and a direction orthogonal to the front-rear direction and the height direction as a width direction. The front side in the front-rear direction is the same direction as the direction in which the injection needle of the syringe housed in the housing 2 is directed.

The receiving part 21 is formed in the opening H of the housing body 20 and curved to protrude inside of the housing 2 (toward the lower side in the height direction). That is, the receiving part 21 has a recessed shape.

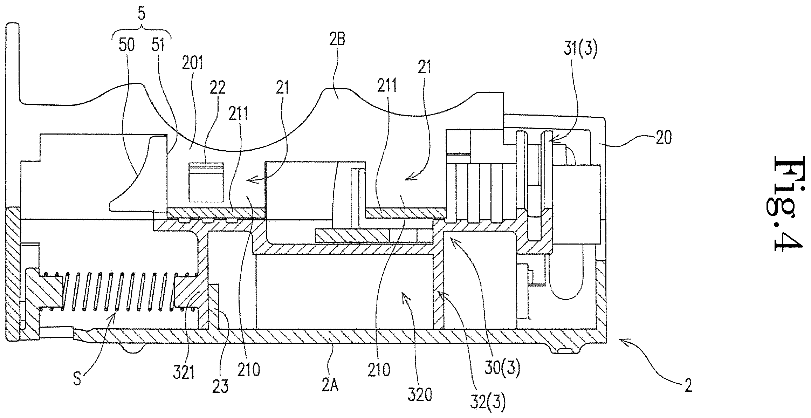

The receiving part 21 according to this embodiment has a pair of lateral parts 210 facing each other in the width direction and a bottom part 211 continuous with each of the pair of lateral parts 210 as shown in Fig. 4. Each of the pair of lateral parts 210 extends straight downward from an opening edge 201 of the housing body 20, and the bottom part 211 connects the ends of the pair of lateral parts 210 with each other.

In this embodiment, two receiving parts 21 are formed with a distance therebetween in the front-rear direction, and a coming-off prevention part 22 is formed on one of the two receiving parts 21 (the receiving part 21 on the front side).

The coming-off prevention part 22 is formed on the inner surface of each one of the lateral parts 210 and has a shape protruding toward the opposite lateral part 210. The coming-off prevention part 22 as an individual part is formed on each of the pair of lateral parts 210. The coming-off prevention part 22 is formed between the opening edge 201 and the bottom part 211 of the housing body 20, and thus configured to come into pressure-contact with an outer surface of the syringe mounted on the bottom part 211 between the opening edge 201 and the bottom part 211 in the housing body 20. Thereby, the syringe is prevented from coming off from the housing body 20.

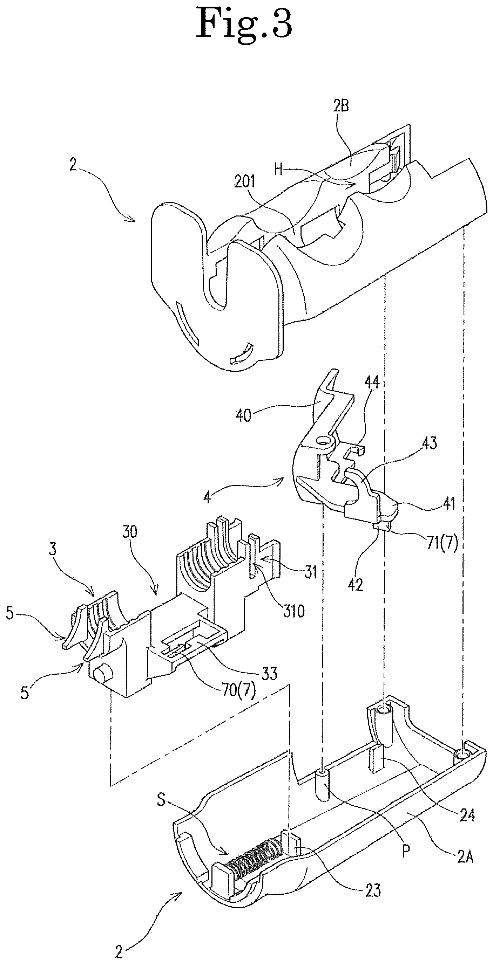

As shown in Fig. 3, the moving part 3 includes a moving part body 30 that is slidable in the housing body in the front-rear direction, a fixing part 31 that fixes the syringe relative to the moving part body 30, and a positioning part 32 for positioning the moving part body 30 at a retracted position to be described later. The moving part 3 is biased rearward in the front-rear direction by a biasing means S arranged in the housing body 20.

In this embodiment, a compression coil spring as the biasing means S is arranged between a front surface (i.e., a front surface of a front wall part 321 to be described later) and an inner end on the distal end side of the housing body 20 so that the moving part 3 is biased rearward in the front-rear direction by a biasing force of the biasing means S.

The fixing part 31 is configured to move the syringe in the front-rear direction in association with the sliding of the moving part 3. The fixing part 31 is engageable with a front face and a rear face of a flange of the syringe. The fixing part 31 according to this embodiment is configured to form a fitting groove 310 into which the flange of the syringe can be fitted.

Therefore, the fixing part 31 is configured to be capable of moving the syringe in the front-rear direction during the sliding of the moving part 3, while restricting the syringe from moving in the front-rear direction with respect to the moving part 3. Thereby, the moving part 3 can change its position, in association with the sliding, between an advanced position at which the injection needle of the syringe is located forward of the contact surface 200 and a retracted position at which the injection needle is located rearward of the contact surface 200.

The positioning part 32 is disposed on the lower side (i.e., on the side close to the case part 2A) of the moving part body 30. A recessed part 320 opening toward the lower side (toward the side close to the case part 2A) is formed in the positioning part 32 and extends along the front-rear direction.

Further, a rising part 23 formed to rise from an inner bottom surface (in the case part 2A) of the housing 2 is inserted in the recessed part 320. When the moving part 3 slides in the front-rear direction and is arranged at the retracted position, the rear surface (the inner surface) of the front wall part 321 comes into contact with the rising part 23. With this, the moving part 3 is configured to be unable to move rearward of the retracted position when the moving part 3 is arranged at the retracted position. Accordingly, the retracted position of the moving part 3 (the position to which the syringe is retracted after an injection solution is administered) is determined by a positioning structure formed by the front wall part 321 (the rear surface of the front wall part 321) and the rising part 23.

As shown in Fig. 5, the operation member 4 includes an operation member body 40 that is pivotally attached to the housing 2, an activating part 41 that extends from the operation member body 40 and moves in the front-rear direction in association with the pivotal movement of the operation member body 40, a pressing part 42 disposed in the activating part 41 to press forward the moving part 3 (i.e., a pressing operation engagement part of the moving part 3 that is pressed forward by the operation member 4), and an anti-drop part 43 provided to prevent the syringe from dropping off from the housing body 20.

The operation member body 40 is capable of pivoting around a pivot axis P extending along an attaching and detaching direction in the housing.

The operation member body 40 is formed by a lever extending outward from the housing 2 and is capable of switching between a raised position at which the lever is raised with respect to the housing 2 and a lowered position at which the lever is lowered with respect to the housing 2. Herein, the raised position corresponds to a first position before the operation member 4 slides the moving part 3 to the advanced position, and the lowered position corresponds to a second position after the operation member 4 has slid the moving part 3 to the retracted position.

The operation member body 40 is configured to restrict switching from the second position to the first position after switching from the first position to the second position. In this embodiment, a locking claw 44 formed in the operation member body 40 engages with a locking shaft 24 formed in the housing 2 when the operation member body 40 switches from the first position to the second position so that the switching from the second position to the first position is restricted. Meanwhile, the operation member body 40 is configured to be able to return to the first position if it is in a state before switching to the second position.

The pressing part 42 and the pressing operation engaging part 33 are in engagement with each other when the operation member body 40 is located at the first position, and the pressing part 42 and the engagement part 33 disengage with each other when the operation member body 40 switches from the first position to the second position.

Similar to the operation member body 40, the activating part 41 is also capable of pivoting around the pivot axis P and thus moves along a circular orbit. That is, the activating part 41 moves with its position changing in the width direction, while moving in the front-rear direction.

The anti-drop part 43 is formed to rise from the activating part 41 and configured to switch between a position at which the anti-drop part 43 advances into the opening H and a position at which the anti-drop part 43 retracts from the opening H in association with the pivoting operation of the operation member 4.

When the anti-drop part 43 advances into the opening H with the syringe housed in the housing body 20, the upper part of the syringe is blocked by the anti-drop part 43 to thereby prevent the syringe from dropping off from the housing body 20 and block the detaching of the syringe from the housing body 20.

The pressing part 42 extends downward from the activating part 41. Further, the pressing part 42 is configured to engage with the moving part 3 (i.e., the pressing operation engaging part 33 of the moving part 3) from the rear side when the operation member body 40 is located at the first position, and to press forward the pressing operation engaging part 33 when the operation member body 40 is switching from the first position to the second position.

Further, since the pressing part 42 has its position changing in the width direction while moving together with the activating part 41 in the front-rear direction, the pressing part 42 is located at a position displaced from the pressing operation engaging part 33 in the width direction when the operation member body 40 switches from the first position to the second position. Thereby, the pressing part 42 is released from the engagement with the engagement part 33.

The separation structure 5 is disposed on the distal end side of the moving part 3 as shown in Fig. 4. The separation structure 5 is formed with a dimension increasing in the front-rear direction from the top part to the bottom side, of the housing 2. In this embodiment, a pair of separation structures 5 are disposed with a distance from each other in the width direction to allow the syringe to pass therebetween.

Each of the separation structures 5 has a guide surface 50 located on the front side and a stop surface 51 located on the rear side. Contrary to the stop surface 51 extending straight in the vertical direction, the guide surface 50 is inclined forward as it advances from the top part of the housing 2 to the bottom part of the housing 2, while being curved downward to have a recessed shape.

Here, the cap includes a seal material disposed inside thereof, into which the injection needle is pierced. Thus, in the case where the cap is removed from the syringe, the cap is detached from the syringe when the seal material moves forward of the distal end of the injection needle (i.e., when the injection needle is pulled out of the seal material) as the syringe advances. Therefore, the dimension of the separation structure in the front-rear direction, more specifically, the dimension from the stop surface 51 to the distal end of the guide surface 50 in the front-rear direction is preferably set to be substantially equivalent to the moving distance required to cause the seal material in the state of being attached to the syringe to be separated from the injection needle.

In the case where the dimension from the stop surface 51 to the distal end of the guide surface 50 in the front-rear direction is shorter than the moving distance, the cap is not detached from the syringe since the cap is unable to sufficiently advance toward the syringe when the capped syringe is inserted into the housing body 20.

In the case where the dimension from the stop surface 51 to the distal end of the guide surface 50 in the front-rear direction is longer than the moving distance, the cap remains in the separation structure 5 even after the seal material is detached from the injection needle, which causes a downward external force to continuously apply to the syringe remaining in this state (that is, to continuously apply to the receiving part 21 side). If the injection needle interferes with the cap in this state, the injection needle may be bent.

In order to deal with this situation, the dimension from the stop surface 51 to the distal end of the guide surface 50 in the front-rear direction is set to be substantially equivalent to the moving distance. With this, the cap drops off from the separation structure 5 when the seal material is separated from the injection needle. Thus, it is possible to prevent the injection needle from interfering with the cap even if the syringe keeps moving downward.

The separation structure 5 is configured so that, when the syringe is inserted into the housing body 20 from the opening H, the guide surface 50 comes into contact with the rear end of the cap from the rear side and the stop surface 51 comes into contact with the shoulder of the syringe from the front side.

As shown in Fig. 2, the restriction structure 6 includes a restricting part 60 disposed at the rear end of the moving part 3, and a restriction engaging part 61 that engages with the restriction part 60 on the rear end side when the moving part 3 is arranged at the advanced position. In the restriction structure 6 according to this embodiment, a pair of restricting parts 60 are disposed with a distance from each other in the width direction, and a pair of engagement parts 61 are respectively disposed outside the pair of the restricting parts 60 (i.e., outside in the width direction).

The restricting part 60 has a straight portion 600 extending rearward from the rear end of the moving part 3 and a protruding portion 601 formed to protrude outward of the straight portion 600 in the width direction.

The restriction engaging part 61 has a restricting extension 610 connected with the housing 2 and extending in the front-rear direction, and a restricting hook portion 611 formed at a distal end of the extension (i.e., one end on the rear side).

The restricting extension 610 has a flexibility and formed to bend in the width direction.

The restricting hook portion 611 protrudes inward in the width direction from the restricting extension 610 and has an inner end (i.e., a distal end in the width direction) located inside of the outer end of the restricting part 60 in the width direction. With this, the restricting hook portion 611 engages with the rear end surface (i.e., a rear end surface of the protruding portion 601) in the front-rear direction. The distal end surface of the restricting hook portion 611 and the rear end surface of the restricting part 60 are parallel to each other. Further, the rear end surface of the restricting hook portion 611 is tapered outward in the width direction as it advances toward the rear side. Therefore, when the rear end surface of the restriction engaging part 61 receives an external force directed forward, the restricting extension 610 bends outward to thereby cause the restricting hook portion 611 to move outward in the width direction. As described above, the restricting hook portion 611 is configured to move outward in the width direction to be released from the engagement with the rear end surface of the protruding portion 601, when the rear end surface of the restricting hook portion 611 receives the external force while in engagement with the rear end surface of the protruding portion 601.

As shown in Fig. 5, the blocking structure 7 includes a block engaging part 70 that is disposed in the moving part 3, and a blocking part 71 that is disposed in the activating part 41 of the operation member 4 and is configured to allow the block engaging part 70 to engage therewith when the moving part 3 moves from the advanced position to the retracted position.

The block engaging part 70 has a blocking extension 700 extending from the moving part 3 along the front-rear direction and a blocking hook portion 701 disposed at the distal end (i.e., one end on the rear side) of the blocking extension 700.

The blocking extension 700 has a flexibility and is configured to bend in the width direction.

The blocking hook portion 701 protrudes inward in the width direction from the blocking extension 700 and is configured to be engaged with the rear end surface of the pressing part 42 from the lateral side of the pressing part 42. The rear end surface of the blocking hook portion 701 is tapered outward in the width direction as it advances toward the rear side and thus bends outward when the rear end surface of the blocking hook portion 701 receives the external force directed forward.

The blocking part 71 and the block engaging part 70 come out of engagement with each other when the operation member body 40 is located at the first position, and the blocking part 71 and the block engaging part 70 come into engagement with each other when the operation member body 40 switches from the first position to the second position, which causes the restriction by the restriction structure 6 to be released and hence causes the moving part 4 to return from the advanced position to the retracted position by a biasing force of the biasing means S.

In the syringe auxiliary tool 1 according to this embodiment, a connecting part is disposed in the moving part to connect the moving part 3 with the operation member 4 and this connecting part is formed by the pressing operation engagement part 33 and the block engaging part 70.

The syringe auxiliary tool 1 according to this embodiment is as described above. Subsequently, the description will be given for the usage of the syringe auxiliary tool 1.

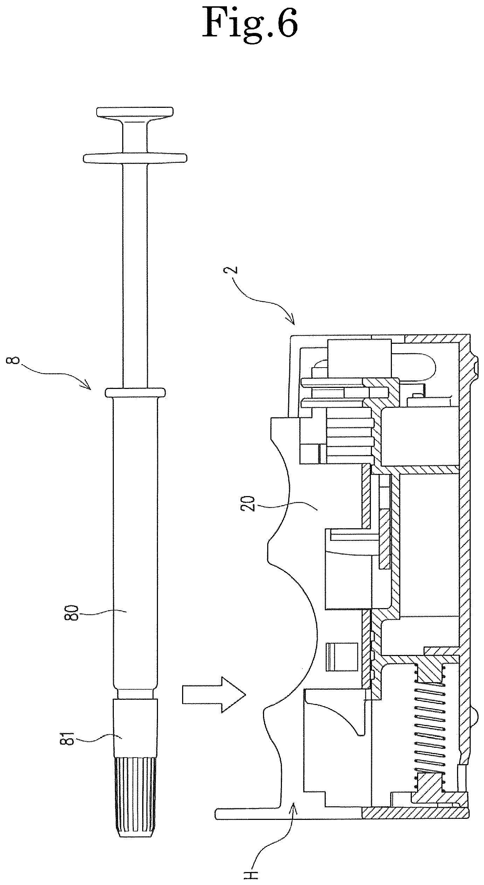

The capped syringe 8 is housed in the housing 2 when an injection is performed using the syringe auxiliary tool 1. In this embodiment, the capped syringe 8, which is laid sideways, is inserted in the housing body 20 from the opening H as shown in Fig. 6.

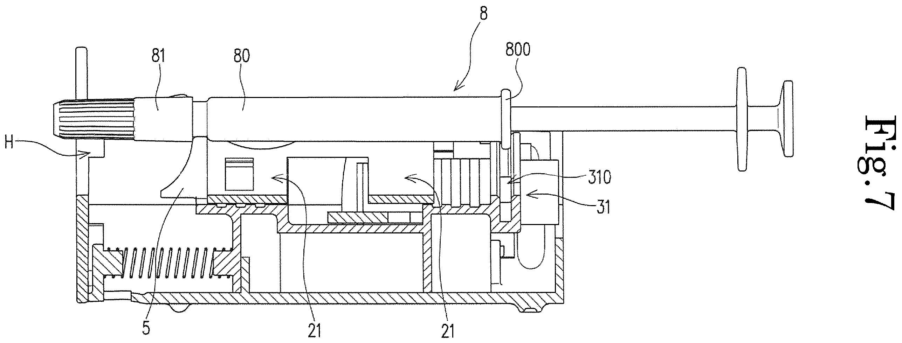

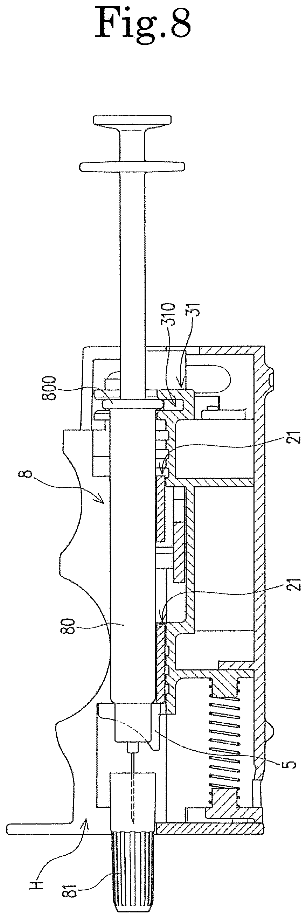

At this time, the separation structure 5 separates a syringe 80 and a cap 81 from each other before the capped syringe 8, which passes through the opening H, reaches the receiving part 21 as shown in Fig. 7 and Fig. 8. Although the cap 81 is guided to the front side by the guide surface 50 of the separation structure 5, the movement in the front-rear direction of the syringe 80 with respect to the moving part 3 is restricted since a flange 800 of the syringe 80 is fitted into a fitting groove 310. Further, a user has no risk of contact with an injection needle 801 since the injection needle 801 is held retracted rearward of the contact surface 200.

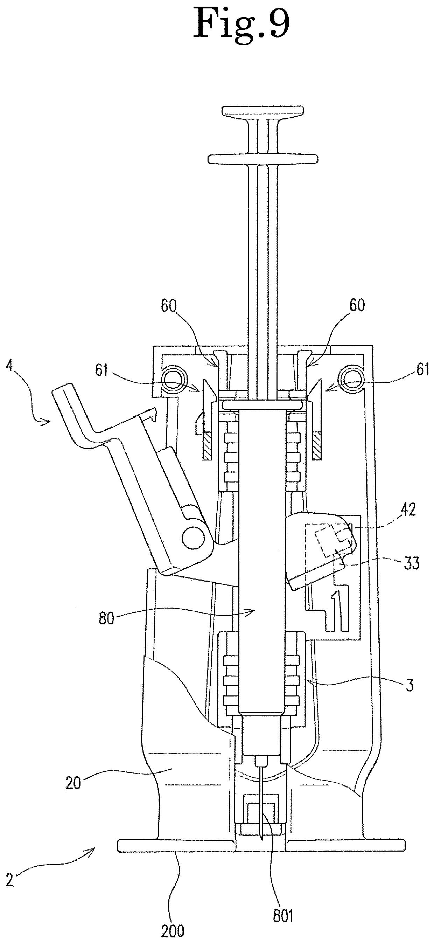

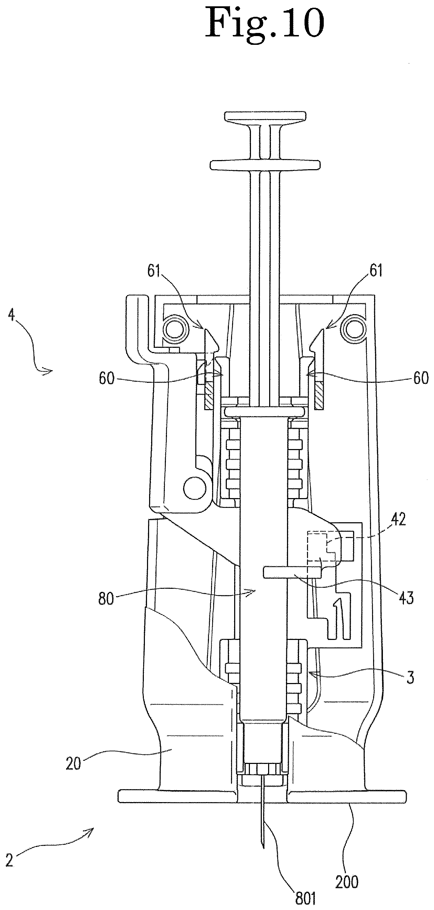

When the injection needle 801 is pierced into a living body, the user switches the operation member 4 from the first position to the second position while forcing the contact surface 200 against the living body as shown in Fig. 9 and Fig. 10. This causes the pressing part 42 to press the moving part 3 forward to thereby cause the syringe to advance forward to cause the injection needle 801 to move forward of the contact surface 200. That is, the injection needle 801 is pierced into the living body. Further, the user can adjust the advancing speed of the injection needle 801 according to the way of pressing of the operation member 4. Further, if the user stops the operation on the operation member 4 in a stage before the operation member 4 switches into the second position, the moving part 3 moves rearward by being pushed back by the biasing means S to cause the injection needle 801 to be automatically retracted into the housing 2. At this time, the pressing part 42 is pushed back to the rear side by the pressing operation engaging part 33, and thus the operation member 4 automatically switches from the first position to the second position.

When the user switches the operation member 4 from the first position to the second position (that is, the user moves forward the moving part 3 from the retracted position to the advanced position), the restricting part 60 and the restriction engaging part 61 engage with each other to thereby restrict the moving part 3 from moving rearward.

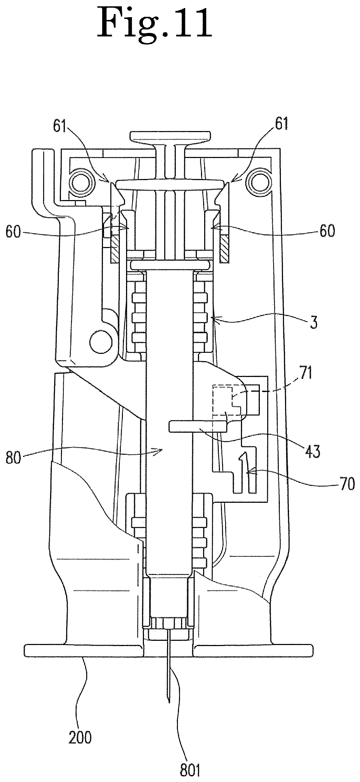

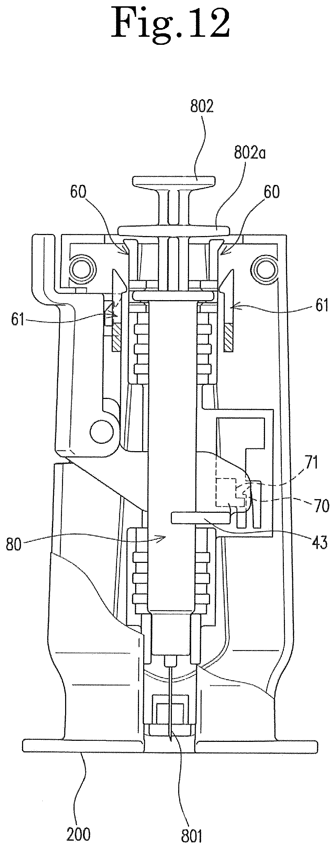

When a plunger 802 is pushed into the syringe, the restriction engaging part 61 is pressed forward from the rear side by the plunger 802 (in this embodiment, a flange 802a formed at a position displaced forward from the rear end of the plunger 802) and expands outward in the width direction to thereby release the restricting part 60 and the restriction engaging part 61 from the engagement with each other as shown in Fig. 11 and Fig. 12. When the user releases his or her hand from the plunger 802, the syringe 80 automatically moves rearward together with the moving part 3. Thereby, the injection needle 801 is retracted rearward of the contact surface 200 so that the injection needle 801 is pulled out from the living body.

At this time, the block engaging part 70 engages with the blocking part 71, the moving part 3 and the syringe 80 are prevented from further advancing, and the upper part of the syringe 80 is held by the anti-drop part 43. Thus, the syringe 80 housed in the housing 2 cannot be detached therefrom while the injection needle 801 is kept retracted in the housing 2.

As described above, according to the syringe auxiliary tool 1 of this embodiment, the user who performs an injection operates the operation member 4 while forcing the contact surface 200 of the housing 2 against the living body to cause the moving part 3 to slide from the retracted position to the advanced position to thereby cause the syringe 80 to move forward together with the moving part 3. Thereby, the injection needle 801 moves forward of the contact surface 200 to cause the injection needle 801 to be pierced into the living body.

Further, according to the syringe auxiliary tool 1 having the aforementioned configuration, the moving part 3 is biased toward the retracting position by the biasing means S. Thus, when the user stops the operation of the operation member 4 in the state where the injection needle 801 is pierced into the living body, the moving part is caused to slide to the retracted position by the biasing force of the biasing means S so that the injection needle 801 is pulled out from the living body.

As described above, according to the syringe auxiliary tool 1 having the aforementioned configuration, the user can stably pierce the injection needle 801 into the living body while forcing the contact surface 200 against the living body. Further, the injection needle 801 is pulled out from the living body by stopping the operation of the operation member 4, even in the case where the injection needle 801 is accidentally pierced. Thus, improved safety during piercing the injection needle 801 can be realized.

According to the syringe auxiliary tool 1 of the present invention, when the user finds an accidental piercing of the injection needle 801 and stops the operation, the injection needle 801 is pulled out from the living body. Thus, it is possible to produce an excellent advantage that the safety during piercing the injection needle 801 can be improved.

Further, according to the syringe auxiliary tool 1, the operation member 4 is separated from the moving part 3, and the restriction structure 6 restricts the moving part 3 from sliding toward the retracted position in a state where the moving part 3 is located at the advanced position, that is, in the state where the injection needle 801 is pierced into the living body. Thus, it is possible to prevent the injection needle 801 from being pulled out from the living body by the user's erroneous operation of the operation member 4 at an unintentional timing while the injection needle 801 is being pierced into the living body.

Further, according to the syringe auxiliary tool 1, the pressing part 42 and the pressing operation engaging part 33 come out of the engagement with each other in the state after the moving part 3 has slid to the advanced position, that is, after the injection needle 801 has been pierced into the living body. Thus, when the restriction by the restriction structure is released, the moving part 3 and the connecting part slide to the retracted position by the biasing force of the biasing means S. At this time, the moving part 3 is blocked from advancing by the engagement of the blocking part 71 with the block engaging part 70 so that the injection needle 801 can be prevented from being exposed again from the contact surface 200.

Thus, the moving part 3 is blocked from advancing by the engagement between the blocking part 71 and the block engaging part 70 when the moving part 3 is thus located at the retracted position after the injection needle 801 is pierced into the living body as described above. In this regard, when the operation member 4 is located at the first position, that is, located at the retracted position at a time point before the injection needle 801 is pierced into the living body, the pressing part 42 and the pressing operation engaging part 33 are in engagement with each other, while the blocking part 71 and the block engaging part 70 are out of engagement with each other, so that the syringe auxiliary tool 1 having the aforementioned configuration does not block the moving part 3 from advancing at a time point before the injection needle 801 is pierced into the living body.

The syringe auxiliary tool according to the invention is defined by the claims and not limited to the embodiments of the description.

In the aforementioned embodiment, the two receiving parts 21 are formed in the housing body 20, but the present invention is not limited to this configuration. For example, three or more of receiving parts 21 may be formed in the housing body 20, or one receiving part 21 may be formed as long as the syringe can be stabled with its posture laid sideways.

In the aforementioned embodiment, the coming-off prevention part 22 is formed in the front one of the two receiving parts 21, but the present invention is not limited to this configuration. For example, the coming-off prevention part 22 may be formed in the rear one of the two receiving parts 21, or the coming-off prevention part 22 may be formed in each of both of the two receiving parts 21.

In the aforementioned embodiment, the moving part 3 is biased rearward in the front-rear direction by the compression coil spring as the biasing means S arranged between the inner end on the distal end side of the housing body 20 and the moving part 3, but the present invention is not limited to this configuration. The moving part 3 may be, for example, biased rearward in the front-rear direction by an extension spring as the biasing means S arranged between the rear surface of the moving part 3 and the inner end on the rear end side inside the housing body 20.

In the aforementioned embodiment, the operation member 4 is configured to enable the moving part 3 to perform only the pressing operation but is not limited to this configuration. For example, the operation member 4 may be configured to enable the moving part 3 to perform a pulling operation.

### REFERENCE SIGNS LIST

1: Auxiliary tool
2: Housing
2A: Case part
2B: Cover part
3: Moving part
4: Operation member
5: Separation structure
6: Restriction structure
7: Blocking structure
8: Capped syringe
20: Housing body
21: Receiving part
22: Coming-off prevention part
23: Rising part
24: Locking shaft
30: Moving part body
31: Fixing part
32: Positioning part
33: Pressing operation engagement part
40: Operation member body
41: Activating part
42: Pressing part
43: Anti-drop portion
44: Locking claw
50: Guide surface
51: Blocking surface
52: Body portion
53: Projection
60: Restricting part
61: Restriction engaging part
70: Block engaging part
71: Blocking part
80: Syringe
81: Cap
200: Contact surface
201: Opening edge
210: Lateral part
211: Bottom part
310: Fitting groove
600: Straight portion
601: Protruding portion
610: Restricting extension
611: Restricting hook portion
700: Blocking extension
701: Blocking hook portion
800: Flange
801: Injection needle
802: Plunger
803: Nozzle
820a: Flange
H: Opening
P: Pivot axis
S: Biasing means

## Claims

1. A syringe auxiliary tool (1) comprising:
a housing (2) that has a contact surface (200) that comes into contact with a living body and houses a syringe (80) with an injection needle (801) directed forward;
a moving part (3) that engages with the syringe (80) housed in the housing (2) and attached to the housing (2) slidably in a front-rear direction, the moving part (3) being capable of changing its position, in association with the sliding, between an advanced position at which the injection needle (801) of the syringe is located forward of the contact surface (200) and a retracted position at which the injection needle (801) is located rearward of the contact surface (200);
and
an operation member (4) that is configured to slide the moving part (3) in a forward direction from the retracted position toward the advanced position, wherein
the syringe auxiliary tool (1) further comprises a biasing means (S) that biases the moving part (3) in a retracting direction from the advanced position toward the retracted position,
the operating member (4) comprises:
an operating member body (40) that is pivotally attached to the housing (2);
an activating part (41) that extends from the operation member body (40) and moves in the front rear direction in association with the pivotal movement of the operation member body (40); and
a pressing part (42) disposed in the activating part (41),
the operating member body (40) is formed by a lever extending outward from the housing (2) and is capable of, in association with the pivotal movement, switching between a raised position at which the lever is raised with respect to the housing (2) and a lowered position at which the lever is lowered with respect to the housing (2),
the pressing part (42) is configured to move forward in the front-rear direction in association with the switching of the operation member body (40) from the raised position to the lowered position, and is configured to move forward in the front-rear direction to thereby press forward the moving part (3) to the advanced position.

2. The syringe auxiliary tool (1) according to claim 1, further comprising:
_a blocking structure (7) that is capable of blocking the syringe (80) from moving forward in the state where the injection needle (801) is retracted in the housing (2), wherein
the moving part (3) comprises a pressing operation engaging part (33) that is pressed forward in the front-rear direction by the operation member (4), and
the pressing part (42) is configured:
to engage with the pressing operation engaging part (33) from the rear side in the front-rear direction when the operation member body (40) is located at the raised position;
to press forward the pressing operation engaging part (33) when the operation member body (40) is switching from the raised position to the lowered position; and
to be released from the engagement with the pressing operation engaging part (33) when the operation member body (40) switches from the raised position to the lowered position.

3. The syringe auxiliary tool (1) according to claim 2,
comprising the blocking structure (7) that is configured to block the moving part (3) from moving forward,
the blocking structure (7) comprising:
a block engaging part (70) that is disposed in the moving part (3); and
a blocking part (71) that is disposed in the activating part (41) and is configured to allow the block engaging part (70) to engage with the blocking part (71) when the moving part (3) moves from the advanced position to the retracted position, wherein
the syringe auxiliary tool (1) is configured such that:
the pressing part (42) and the pressing operation engaging part are (33) in engagement with each other while the blocking part (71) and the block engaging part (70) are out of engagement with each other when the operation member (4) is located at the raised position; and
when the operation member (4) switches from the raised position to the lowered position, the blocking part (71) and the block engaging part (70) engage with each other by the moving part (3) and the block engaging part (70) returning from the advanced position to the retracted position by a biasing force of the biasing means (S).

## Patentansprüche

1. Spritzenhilfswerkzeug (1), umfassend:
ein Gehäuse (2), das eine Kontaktfläche (200) aufweist, die mit einem lebenden Körper in Kontakt kommt und eine Spritze (80) mit einer nach vorne gerichteten Injektionsnadel (801) aufnimmt;
ein bewegliches Teil (3), das mit der in dem Gehäuse (2) aufgenommenen Spritze (80) in Eingriff steht und an dem Gehäuse (2) in einer Vorwärts-Rückwärts-Richtung verschiebbar angebracht ist, wobei das bewegliche Teil (3) in der Lage ist, seine Position in Verbindung mit dem Verschieben zwischen einer vorgeschobenen Position, in der sich die Injektionsnadel (801) der Spritze vor der Kontaktfläche (200) befindet, und einer zurückgezogenen Position, in der sich die Injektionsnadel (801) hinter der Kontaktfläche (200) befindet, zu ändern;
und
ein Betätigungselement (4), das konfiguriert ist, um das bewegliche Teil (3) in einer Vorwärtsrichtung von der zurückgezogenen Position zu der vorgeschobenen Position zu verschieben, wobei
das Spritzenhilfswerkzeug (1) ferner ein Vorspannmittel (S) umfasst, das das bewegliche Teil (3) in einer Rückzugsrichtung von der vorgeschobenen Position zu der zurückgezogenen Position vorspannt,
das Betätigungselement (4) umfasst:
einen Betätigungselementkörper (40), der schwenkbar an dem Gehäuse (2) angebracht ist;
ein Aktivierungsteil (41), das sich von dem Betätigungselementkörper (40) erstreckt und sich in Verbindung mit der Schwenkbewegung des Betätigungselementkörpers (40) in der Vorwärts-Rückwärts-Richtung bewegt; und
ein Druckteil (42), das in dem Aktivierungsteil (41) angeordnet ist,
der Betätigungselementkörper (40) durch einen Hebel gebildet ist, der sich von dem Gehäuse (2) nach außen erstreckt und in der Lage ist, in Verbindung mit der Schwenkbewegung zwischen einer angehobenen Position, in der der Hebel in Bezug auf das Gehäuse (2) angehoben ist, und einer abgesenkten Position, in der der Hebel in Bezug auf das Gehäuse (2) abgesenkt ist, umzuschalten,
das Druckteil (42) konfiguriert ist, um sich in Verbindung mit dem Umschalten des Betätigungselementkörpers (40) von der angehobenen Position zu der abgesenkten Position in der Vorwärts-Rückwärts-Richtung vorwärts zu bewegen, und konfiguriert ist, um sich in der Vorwärts-Rückwärts-Richtung vorwärts zu bewegen, um dadurch das bewegliche Teil (3) zu der vorgeschobenen Position vorwärts zu drücken.

2. Spritzenhilfswerkzeug (1) nach Anspruch 1, ferner umfassend:
_eine Blockierstruktur (7), die in der Lage ist, die Spritze (80) in dem Zustand, in dem die Injektionsnadel (801) in das Gehäuse (2) zurückgezogen ist, daran zu hindern, sich vorwärts zu bewegen, wobei
das bewegliche Teil (3) ein Druckbetätigungseingriffsteil (33) umfasst, das durch das Betätigungselement (4) in der Vorwärts-Rückwärts-Richtung vorwärts gedrückt wird, und
das Druckteil (42) konfiguriert ist:
um mit dem Druckbetätigungseingriffsteil (33) von der Rückseite in der Vorwärts-Rückwärts-Richtung in Eingriff zu kommen, wenn sich der Betätigungselementkörper (40) in der angehobenen Position befindet;
um das Druckbetätigungseingriffsteil (33) vorwärts zu drücken, wenn der Betätigungselementkörper (40) von der angehobenen Position zu der abgesenkten Position umschaltet; und
um aus dem Eingriff mit dem Druckbetätigungseingriffsteil (33) gelöst zu werden, wenn der Betätigungselementkörper (40) von der angehobenen Position zu der abgesenkten Position umschaltet.

3. Spritzenhilfswerkzeug (1) nach Anspruch 2,
umfassend die Blockierstruktur (7), die konfiguriert ist, um das bewegliche Teil (3) daran zu hindern, sich vorwärts zu bewegen,
wobei die Blockierstruktur (7) umfasst:
ein Blockiereingriffsteil (70), das in dem beweglichen Teil (3) angeordnet ist; und
ein Blockierteil (71), das in dem Aktivierungsteil (41) angeordnet ist und konfiguriert ist, um es dem Blockiereingriffsteil (70) zu ermöglichen, mit dem Blockierteil (71) in Eingriff zu kommen, wenn sich das bewegliche Teil (3) von der vorgeschobenen Position zu der zurückgezogenen Position bewegt, wobei
das Spritzenhilfswerkzeug (1) derart konfiguriert ist, dass:
das Druckteil (42) und das Druckbetätigungseingriffsteil (33) miteinander in Eingriff stehen, während das Blockierteil (71) und das Blockiereingriffsteil (70) nicht miteinander in Eingriff stehen, wenn sich das Betätigungselement (4) in der angehobenen Position befindet; und
wenn das Betätigungselement (4) von der angehobenen Position zu der abgesenkten Position umschaltet, das Blockierteil (71) und das Blockiereingriffsteil (70) miteinander in Eingriff kommen, indem das bewegliche Teil (3) und das Blockiereingriffsteil (70) durch eine Vorspannkraft des Vorspannmittels (S) von der vorgeschobenen Position zu der zurückgezogenen Position zurückkehren.

## Revendications

1. Outil auxiliaire pour seringue (1) comprenant :
un boîtier (2) comportant une surface de contact (200) qui entre en contact avec un corps vivant et qui reçoit une seringue (80) avec une aiguille d'injection (801) dirigée vers l'avant ;
un élément mobile (3) qui s'engage avec la seringue (80) logée dans le boîtier (2) et qui est monté de manière coulissante sur le boîtier (2) dans une direction avant-arrière, l'élément mobile (3) étant capable de changer de position en fonction du coulissement entre une position avancée dans laquelle l'aiguille d'injection (801) de la seringue se trouve devant la surface de contact (200), et une position rétractée dans laquelle l'aiguille d'injection (801) se trouve derrière la surface de contact (200) ;
et
un élément d'actionnement (4) configuré pour déplacer l'élément mobile (3) dans une direction vers l'avant depuis la position rétractée vers la position avancée, dans lequel
l'outil auxiliaire de seringue (1) comprend en outre un moyen de précontrainte (S) qui précontraint la partie mobile (3) dans une direction de retrait de la position avancée vers la position rétractée,
l'élément d'actionnement (4) comprenant :
un corps d'élément d'actionnement (40) monté de manière pivotante sur le boîtier (2) ;
une partie d'activation (41) s'étendant à partir du corps d'élément d'actionnement (40) et se déplaçant dans la direction avant-arrière en association avec le mouvement de pivotement du corps d'élément d'actionnement (40) ; et
une partie de pression (42) disposée dans la partie d'activation (41),
le corps d'élément d'actionnement (40) est formé par un levier qui s'étend vers l'extérieur à partir du boîtier (2) et qui est capable, en liaison avec le mouvement de pivotement, de basculer entre une position relevée dans laquelle le levier est relevé par rapport au boîtier (2) et une position abaissée dans laquelle le levier est abaissé par rapport au boîtier (2) .
la partie de pression (42) est configurée pour se déplacer vers l'avant dans la direction avant-arrière en liaison avec le passage du corps d'élément d'actionnement (40) de la position relevée à la position abaissée, et est configurée pour se déplacer vers l'avant dans la direction avant-arrière, afin de pousser ainsi la partie mobile (3) vers la position avancée.

2. Outil auxiliaire pour seringue (1) selon la revendication 1, comprenant en outre :
une structure de blocage (7) capable d'empêcher la seringue (80) de se déplacer vers l'avant dans l'état où l'aiguille d'injection (801) est rétractée dans le boîtier (2), dans lequel
la partie mobile (3) comprend une partie d'engagement d'actionnement par pression (33) qui est poussée vers l'avant dans la direction avant-arrière par l'élément d'actionnement (4), et
la partie de pression (42) est configurée
pour s'engager avec l'élément d'engagement à pression (33) depuis l'arrière dans la direction avant-arrière lorsque le corps de l'élément d'actionnement (40) se trouve dans la position relevée ;
pour pousser la partie d'engagement d'actionnement par pression (33) vers l'avant lorsque le corps de l'élément d'actionnement (40) passe de la position relevée à la position abaissée ; et
pour être désengagée de la partie d'engagement actionnée par pression (33) lorsque le corps d'élément d'actionnement (40) passe de la position relevée à la position abaissée.

3. Outil auxiliaire pour seringue (1) selon la revendication 2,
comprenant la structure de blocage (7) configurée pour empêcher la partie mobile (3) de se déplacer vers l'avant,
la structure de blocage (7) comprenant :
une partie d'engagement de blocage (70) disposée dans la partie mobile (3) ; et
un élément de blocage (71) disposé dans l'élément d'activation (41) et configuré pour permettre à l'élément d'engagement de blocage (70) de s'engager avec l'élément de blocage (71) lorsque l'élément mobile (3) se déplace de la position avancée vers la position rétractée, dans lequel
l'outil auxiliaire pour seringue (1) est configuré de telle sorte que :
l'élément de pression (42) et l'élément d'engagement d'actionnement de pression (33) s'engagent l'un avec l'autre tandis que l'élément de blocage (71) et l'élément d'engagement de blocage (70) ne s'engagent pas l'un avec l'autre lorsque l'élément d'actionnement (4) est dans la position relevée ; et
lorsque l'élément d'actionnement (4) passe de la position relevée à la position abaissée, la partie de blocage (71) et la partie d'engagement de blocage (70) s'engagent l'une avec l'autre en faisant revenir la partie mobile (3) et l'élément d'engagement de blocage (70) reviennent de la position avancée à la position rétractée sous l'effet d'une force de précontrainte du moyen de précontrainte (S).
